# EUROPÄISCHE PATENTSCHRIFT

(11) **EP 0 920 625 B1**
(45) Veröffentlichungstag und Bekanntmachung des Hinweises auf die Patenterteilung: **28.03.2001**
(21) Anmeldenummer: 97935554.2
(22) Anmeldetag: 30.07.1997
(51) Int. Cl.: G01N 33/543

(54) **VERFAHREN ZUM WIRKSTOFF-SCREENING**
ACTIVE SUBSTANCE SCREENING PROCESS
PROCEDE DE CRIBLAGE DE SUBSTANCES ACTIVES

(30) Priorität: 02.08.1996 DE 19631395
(43) Veröffentlichungstag der Anmeldung: 09.06.1999
(73) Patentinhaber: BASF AKTIENGESELLSCHAFT, 67056 Ludwigshafen (DE)
(72) Erfinder: FUCHS, Harald, D-48301 Nottuln (DE); FRIEDRICH, Thomas, D-64283 Darmstadt (DE); SCHREPP, Wolfgang, D-69118 Heidelberg (DE)
(74) Vertreter: Isenbruck, Günter, Dr.
(86) Internationale Anmeldenummer: EP9704152
(87) Internationale Veröffentlichungsnummer: WO9805963

(56) Entgegenhaltungen:
- US-A- 5 363 697
- US-A- 5 479 024
- N.D. COOK: "Scintillation proximity assay: a versatile high-throughput screening technology.", DRUG DISCOVERY TODAY, CARDIFF UK, , Band 1, Nr. 7, Seiten 287 - 293

## Beschreibung

Die Erfindung betrifft ein Verfahren zum Wirkstoff-Screening, insbesondere ein Verfahren zum Massenscreening im Wirkstoffbereich. Des weiteren betrifft die Erfindung die Verwendung einer Nahfeldmikroskopiervorrichtung sowie eine Einrichtung umfassend eine Nahfeldmikroskopiervorrichtung und eine Steuervorrichtung. Außerdem betrifft die Erfindung noch eine Nanotitervorrichtung.

Das Finden neuer Leitstrukturen, d.h. von molekularen Strukturen wie etwa Liganden, Antagonisten oder Wirkstoffen, die bezüglich ihrer Wirkung auf Enzyme oder Rezeptoren, DNA-Nukleasen oder -Polymerasen, Proteasen wie z.B. Thrombin, Membranrezeptoren, Kernrezeptoren oder Bindeproteine verbessert sind, ist auch derzeit noch ein mühsamer und zeitaufwendiger Suchprozeß.

Der Erfolg hängt dabei in großem Ausmaße vom Zufall ab. Denn dabei müssen sehr viele Substanzen durchgemustert werden, bis aktive Substanzen gegen das Zielmolekül - etwa einen Rezeptor - gefunden werden. Viele Substanzen - etwa 100.000 bis 1.000.000 - auf ihre Bioaktivität hin zu überprüfen, dauert sehr lange, wenn man berücksichtigt, daß Aktivitätstests mit z.B. Zellen oder Enzymen lange Inkubationszeiten erfordern. Eine derartig große Substanzzahl kann daher nur dann schnell geprüft und untersucht werden, wenn auch der Test hochempfindlich und sehr schnell ist.

Vielfach werden bei solchen Tests Fluoreszenzverfahren eingesetzt. Diese sind zwar sehr empfindlich, aber auch störanfällig. Außerdem sind derartige Testverfahren auch sehr zeitaufwendig. Man muß nämlich berücksichtigen, daß von der eigentlichen Inkubation bis zum Meßvorgang noch eine Vielzahl weiterer Behandlungsschritte erforderlich ist. Dazu gehören hier vor allem Waschschritte, um die Hintergrundfluoreszenz zu verringern und um so das Signal-/Rauschverhältnis zu verbessern. Generell kann gesagt werden, daß Verbesserungen in der Empfindlichkeit der Detektionsverfahren zu einer Verlangsamung in der Geschwindigkeit eines Tests führen. Eine Lösung beider Probleme zugleich durch ein Testverfahren bzw. eine entsprechende Einrichtung schien bisher nicht möglich.

In jüngster Zeit kam es mit der Entdeckung der Rastertunnelmikroskopie (RTM; engl. STM) durch Binnig und Rohrer [G. Binnig, H. Rohrer, C. Gerber, E. Weibel, Phys. Rev. Lett. 49 (1982), 57] zu einer stürmischen Entwicklung auf dem Gebiet der höchst sensitiven sog. Rastersondenverfahren.

Bei der Rastertunnelmikroskopie wird eine metallische Spitze zeilenweise rasternd über eine leitende oder halbleitende Probe geführt. Mit dem bei sehr geringen Abständen zwischen Spitze und Probe auftretenden Tunnelstrom als Regelgröße läßt sich ein Höhenprofil und/oder Profil der elektronischen Struktur der untersuchten Oberfläche aufnehmen. Auf diese Weise lassen sich Strukturen mit einer atomaren Auflösung von etwa 0,1 nm unter normalen Laborbedingungen untersuchen. Im Jahre 1986 [G. Binnig, C.F. Quate, C. Gerber, Phys. Rev. Lett. 56 (1986) 930] erfolgte mit der Rasterkraftmikroskopie (Scanning Force Microscopy, SFM) eine Übertragung auf nichtleitende Proben. Damit wurde die Untersuchung von polymeren Materialien [G. Krausch, M. Hipp, M. Böltau, O. Marti, J. Mlynek, Macromolecules 28 (1995) 260] sowie biologischen Strukturen sogar unter physiologischen Bedingungen möglich [C. Bustamante, D. Keller, Physics Today, December 1995, 32]. Bei der SFM wird eine üblicherweise pyramidale Spitze aus Silicium oder Siliciumnitrid, die sich an einem weichen Hebelarm befindet, zur Abtastung der Probe verwendet. Die Auslenkung des Hebelarms wird in der Regel lichtoptisch gemessen und zur Rekonstruktion des Höhenprofils verwendet.

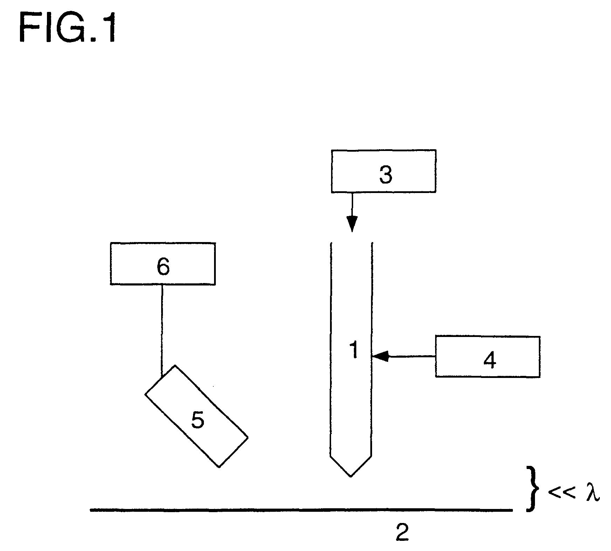

Wird statt der beschriebenen Spitze eine angespitzte Lichtleiterfaser, in die z.B. Laserlicht eingekoppelt wird, als Sonde über die Probe geführt und das je nach Eigenschaft der Probe reflektierte oder transmittierte Licht mit Hilfe von Fotodetektoren nachgewiesen, so gelangt man zur sog. rasteroptischen Nahfeldmikroskopie (Scanning Nearfield Optical Microscopy, SNOM oder auch NSOM genannt) [H. Heinzelmann, D.W. Pohl, Appl. Phys. A 59 (1994) 89; E. Betzig, J. Trautman, Science 257 (1992) 189]. Diese Verfahren erlauben bei Nutzung aller gängigen optischen Kontrastmechanismen wie Absorption, Reflexion, Polarisation, Fluoreszenz usw. durch Umgehung der in der konventionellen Optik auftretenden Beugungsbegrenzung eine sehr hohe Ortsauflösung bis hinunter in den nm-Bereich. Im Bereich der Fluoreszenzmikroskopie wird dabei eine Empfindlichkeit erreicht, die den Nachweis einzelner fluoreszierender Moleküle erlaubt [E. Betzig, R.J. Chichester, Science 262 (1993) 1422]. Ein Schema dieses Verfahrens ist in Fig. 1 angegeben.

Neben der vorstehend stellvertretend beschriebenen SNOM-Anordnung sind im Stand der Technik weitere Anordnungen beschrieben. So können tetrahedrale Spitzen (Koglin J. et al., Photons and Local Probes. Proc. of the NATO Advanced Research Workshop. Ed. Mart O. et al. Kluwer Academic Publ. 1995) oder z.B. photoleitende Spitzen (Akamine S. et al., Proceedings. IEEE Micro Electro Mechanical Systems 1995, Seite 155) verwendet werden.

Die US-A-5,363,697 beschreibt eine rasteroptische Nahfeldmikroskopiervorrichtung (SNOM-Verbindung) sowie eine Mehrfachprobenaufhahmevorrichtung, umfassend eine Platte, die ein Silikonsubstrat mit schachbrettartigem Muster mit unterschiedlich vorbehandelten quadratischen Bereichen von jeweils 2 um Kantenlänge aufweist.

Im biologischen Bereich wurden mittels dieser Techniken erst vereinzelt Untersuchungen durchgeführt, z. B. S. Smith et al., Proc. SPIE-Int. Soc. Opt. Eng. 1855, 81 (1994)(Scanning Probe Microscopies II) oder D.M.N. Jondle et al. Chromosome Research 3, 239 (1995). In der zuletzt genannten Literaturstelle ging es dabei um die Untersuchung der Ultrastruktur polytäner Chromosomen aus der Speicheldrüse der Fruchtfliege mittels SFM.

Unter Berücksichtigung des vorstehend Gesagten ist es Aufgabe der Erfindung, ein Verfahren zum Wirkstoff-Screening bereitzustellen, bei dem eine Vielzahl von Proben in möglichst kurzer Zeit untersucht werden kann. Dieses Verfahren soll nicht nur zeitsparend sein, sondern auch - im Vergleich zu den bislang üblichen Verfahren - möglichst wenig Arbeitsaufwand erfordern.

Ein bekanntes Verfahren zum Wirkstoff-Screeaning ist die Scintillation proximity assay - SPA-Technik, die beispielsweise in Drug Discovery Today 47 (1996), Seiten 287 bis 293 beschrieben ist. Die SPA-Technik hat jedoch den Nachteil, daß die zu untersuchenden chemischen Strukturen zwingend radioaktiv markiert sein müssen, vorzugsweise mit radioaktiven Isotopen des Wasserstoffs oder Jods.

Diese Aufgabe wird durch ein Verfahren gelöst, bei dem man den Wirkstoff über die mittels einer rasteroptischen Nahfeldmikroskopiervorrichtung (SNOM)-Vorrichtung) beobachtbare Änderung eines Signals [S] nachweist. Gegenstand der Erfindung ist also auch die Verwendung einer SNOM-Vorrichtung in Screening-Verfahren sowie eine Einrichtung, umfassend eine Nahfeldmikroskopiervorrichtung, eine Einzel- oder Mehrfachprobenaufnahmevorrichtung und eine Steuereinrichtung zum Ansteuern der einzelnen Kompartimente der Einzel- oder Mehrfachprobenaufbewahrungsvorrichtung mit der SNOM-Vorrichtung.

Bevorzugte Ausgestaltungen der Erfindung sind Gegenstand der Unteransprüche.

Es zeigen:
- Fig. 1: den grundsätzlichen Aufbau einer SNOM-Vorrichtung, unter Verwendung einer Lichtleiterfaser
- Fig.2a) und b): die bei dem erfindungsgemäßen Screening-Verfahren unter Einsatz der SNOM ablaufenden Verfahrensschritte vor [a)] bzw. nach [b)] Zugabe einer den Wirkstoff W₂ und weitere Wirkstoffe W_{X} enthaltenden Lösung zu dem Komplex aus Rezeptor R₁ und fluoreszenzmarkiertem Wirkstoff W₁;
- Fig.3a) und b): die gleichen Verfahrensschritte wie in Fig. 2 a) und b), außer daß die Anordnung von Rezeptor R₁ und Wirkstoff W₁ umgekehrt ist;
- Fig. 4: die bei einer Ausführungsform des erfindungsgemäßen Verfahrens nach Art einer Sandwich-Technik ablaufenden Verfahrensschritte;
- Fig. 5: eine weitere Ausführungsform des erfindungsgemäßen Verfahrens, bei der der unbekannte Wirkstoff W_{X} indirekt über Konformationsänderung des Rezeptors nachgewiesen wird.

Eine SNOM-Vorrichtung, wie sie schematisch in Figur 1 dargestellt ist, weist prinzipiell eine angespitzte Lichtleiterfaser (1) auf, in die z.B. Ar-Laserlicht aus einer Lichtquelle (3) eingekoppelt wird und die mittels einer Steuereinheit (4) über die Probe (2) geführt wird sowie einen Fotodetektor (5) mit Nachweiselektronik (6) für das von der Probe (2) reflektierte oder transmittierte Licht.

Wie bereits erwähnt, ist auch Gegenstand der Erfindung ein Verfahren zum Wirkstoff-Screening, insbesondere im Bereich des Massen-Screenings, bei dem die SNOM-Technologie zum Einsatz kommt. Mit Hilfe dieser Technologie ist es möglich, die Fluoreszenz einzelner Moleküle nachzuweisen, wenn der Abstand zwischen Sonde und Probe im Bereich von einigen nm liegt. Allerdings ist bei dem erfindungsgemäßen Verfahren der Einsatz der SNOM-Technologie grundsätzlich nicht auf den Bereich der Fluoreszenzverfahren beschränkt. Vielmehr kann jedes Nachweisverfahren verwendet werden, bei dem der zu entdeckende neue Wirkstoff eine Änderung eines optischen Signals verursacht. Bei dem optischen Signal kann es sich folglich außer um Fluoreszenz auch um Absorption, Polarisation oder eine Änderung der Wellenlängenabhängigkeit dieser Eigenschaften handeln.

Auch die bei dem erfindungsgemäßen Verfahren eingesetzte SNOM-Technologie ist nicht darauf beschränkt, daß - wie in den Fig. 1, 2 und 3 dargestellt - das optische Nahfeld mittels einer Lichtleiterfaser zur Verfügung gestellt wird. Es sind sämtliche rasteroptische Einrichtungen einsetzbar, solange sie ein Nahfeld bereitstellen, mit dem sich ähnliche Auflösungen erzielen lassen, wie es unter Einsatz einer Lichtleiterfaser möglich ist. Dazu gehören beispielsweise die vorstehend genannten tetrahedralen oder photoleitenden Spitzen.

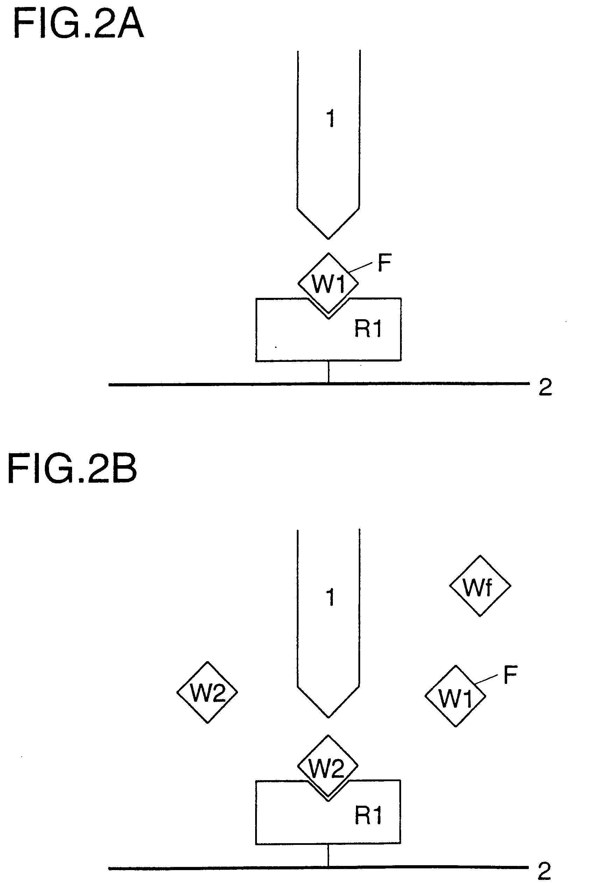

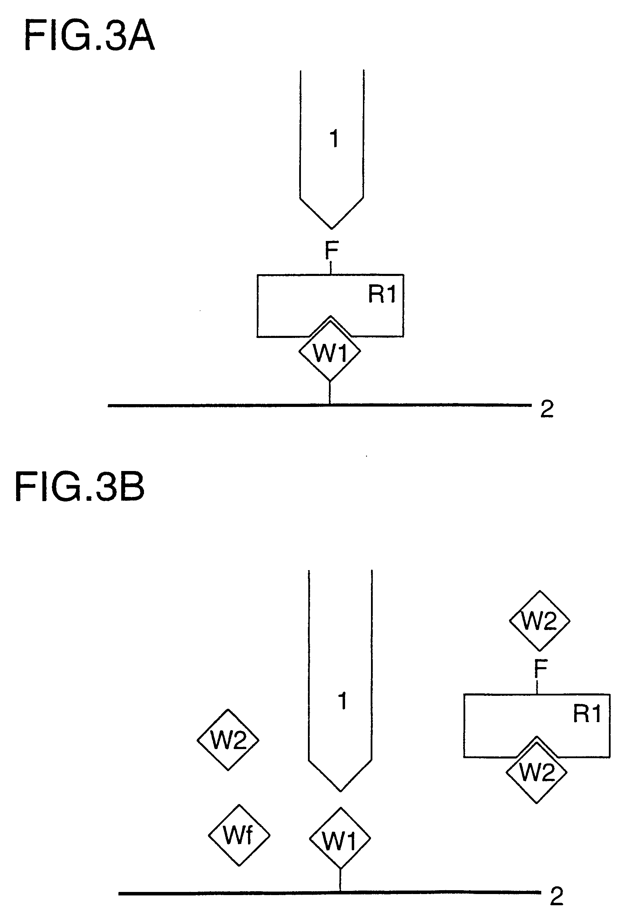

Für das Wirkstoff-Screening läßt sich SNOM beispielsweise vorteilhaft in der folgenden Weise einsetzen:
Zunächst wird ein Rezeptor R1 (Fig. 2) bzw. ein Referenzwirkstoff W1 (Fig. 3) auf einer geeigneten Oberfläche eines Substrats 2 immobilisiert. Der Begriff Rezeptor wird hier sehr umfassend verstanden. Dazu gehören etwa Fibrinogen, Endothelin, DNA oder RNA, Proteine und Lipide im weitesten Sinne, aber auch ganze Zellen oder Gewebestücke. Dieser zu immobilisierende Stoff wird nachfolgend auch als Molekül A bezeichnet. Bei dem Substrat kann es sich um einen mit Gold bedampften Siliciumwafer handeln, eine modifizierte, z.B. silanisierte Siliciumoberfläche oder auch andere ebene Substrate wie etwa Glimmer, Graphit usw. Auch können biologische Substrate eingesetzt werden. Dabei kann es jedoch erforderlich sein, diese zuvor auf den vorstehend genannten anorganischen Substraten zu immobilisieren. Der immobilisierte Rezeptor (R₁) (Fig. 2) oder Referenzwirkstoff (W₁) (Fig. 3) wird mit einem fluoreszenzmarkierten Wirkstoff (W₁-F) bzw. fluoreszenzmarkierten Rezeptor (R₁-F), im folgenden als Molekül B bezeichnet, belegt und sozusagen blockiert. Wird ein stärker bindender Wirkstoff W₂ [Fig. 2 b) bzw. Fig. 3b)] mit höherer Affinität hinzugegeben, so wird der Referenzwirkstoff (W₁) aus der Rezeptorbindungsstelle verdrängt und der Wirkstoff W₂ gebunden. Damit wird der fluoreszenzmarkierte Wirkstoff W₁ von der Oberfläche verdrängt (Fig. 2b). Der gleiche Vorgang tritt im umgekehrten Falle ein, wenn der Referenzwirkstoff W₁ am Substrat fixiert ist und die beiden Wirkstoffe W₁ und W₂ um die Bindung an einem nicht fixierten, aber fluoreszenzmarkierten Rezeptor konkurrieren (Fig. 3). In beiden Fällen wird die durch die Lichtleiterfaser 1, die sich quasi direkt über dem Rezeptor befindet, registrierte Fluoreszenz reduziert oder geht sogar bis auf Null zurück. Sie kann aber auch lediglich moduliert werden, z.B. in ihrer Wellenlängenabhängigkeit geändert werden. In dem Falle ändert sich nicht die Intensität, sondern die Farbe der Fluoreszenz.

Ein besonderer Vorteil des erfindungsgemäßen Verfahrens unter Einsatz der SNOM-Technologie liegt darin, daß fluoreszierende Moleküle W_{f}, die sich aufgrund von Diffusion frei in der Lösung bewegen, praktisch keine Rolle spielen. Sie werden von der SNOM-Sonde nämlich nicht detektiert. Das Volumen, aus dem die relevante Information bei Einsatz der SNOM resultiert, ist aufgrund der starken Abstandsabhängigkeit des Signals extrem klein. Es reicht von einigen nm³ bis zu maximal einigen 10000 nm³. Dadurch spielen frei diffundierende fluoreszierende Moleküle bei diesem Verfahren keine Rolle, so daß sich ein sehr gutes Signal-/Rauschverhältnis ergibt. Dies wiederum hat gegenüber den bislang bekannten hochempfindlichen Meßverfahren den entscheidenden Vorteil, daß eine zeitaufwendige Nachbehandlung der Probe, z.B. durch mehrfaches Waschen, entfällt.

In Anbetracht dessen ist auch klar, daß es bei dem erfindungsgemäßen Verfahren nicht entscheidend ist, daß das Nahfeld durch einen Lichtleiter bereitgestellt wird. Jedes Verfahren, das ein entsprechendes Nahfeld bereitstellt, führt zu den o.g. Vorteilen.

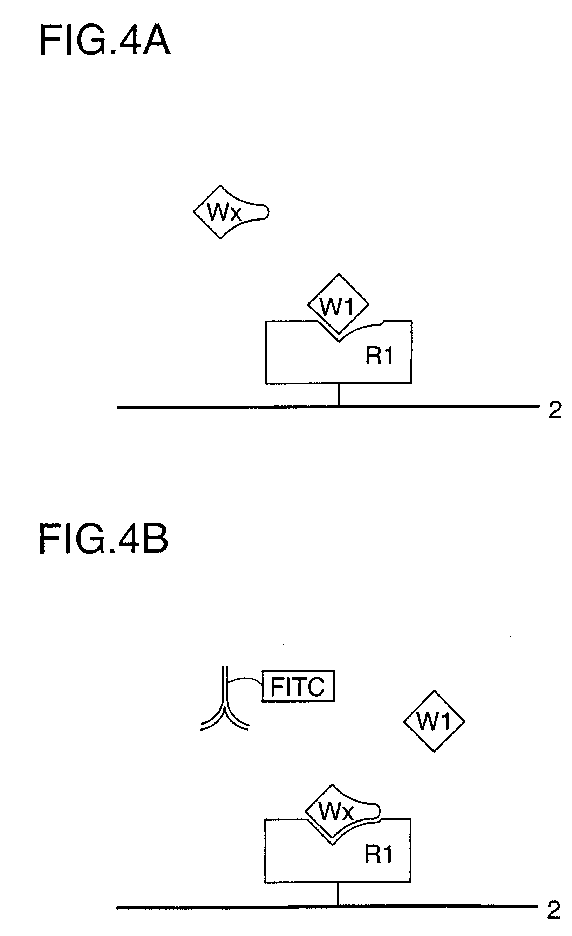

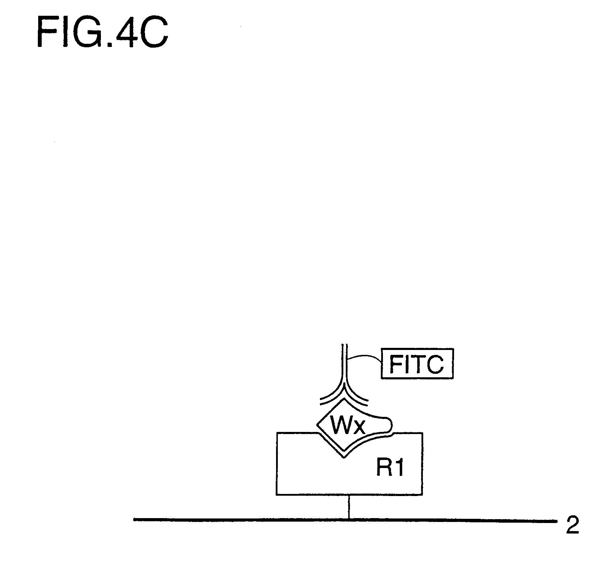

Eine weitere Ausführungsform des erfindungsgemäßen Verfahrens (Sandwich-Technik) ist in Fig. 4 gezeigt. Diese unterscheidet sich von den in den vorstehend erläuterten Fig. 1 bis 3 dargestellten Verfahren dadurch, daß der neue Wirkstoff W_{X} direkt nachgewiesen wird. Zunächst bindet der unbekannte Wirkstoff W_{X} an den Rezeptor R₁. Dieser neue Wirkstoff W_{X} weist eine Domäne (*) auf, von der er sich von dem bekannten Wirkstoff W₁ unterscheidet. Zum Nachweis dieser Domäne steht eine bekannte molekulare Sonde, z.B. ein mit Fluorescein-isothiocyanat (FITC) markierter Antikörper zur Verfügung. Dieser fluoreszenz-markierte Antikörper wird an den Komplex aus Wirkstoff W_{X} und R₁ binden gelassen. Auf diese Weise kann der Komplex unmittelbar über die detektierte Fluoreszenz nachgewiesen werden.

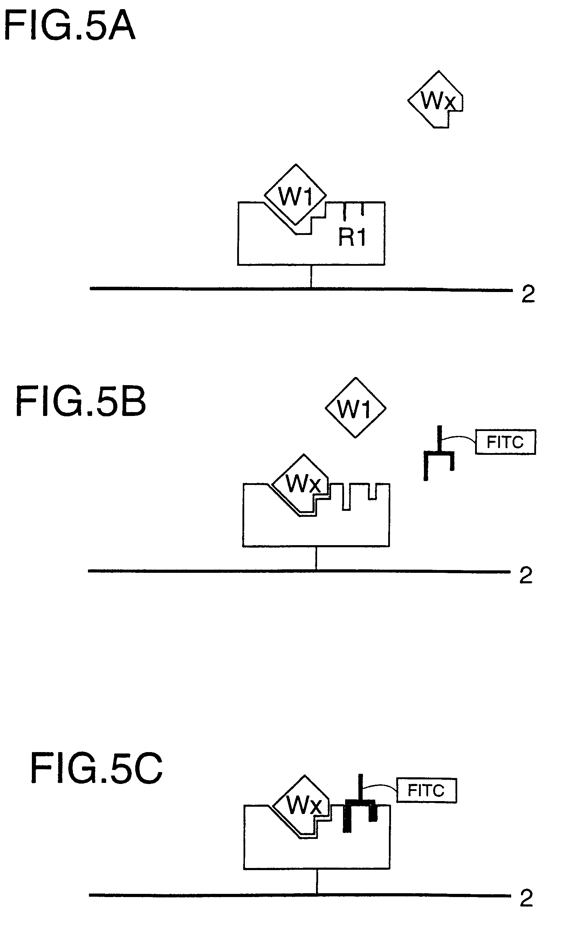

Eine weitere Ausführungsform des erfindungsgemäßen Verfahrens ist in der Fig. 5 dargestellt. Diese Ausführungsform unterscheidet sich von der in Fig. 4 dargestellten dadurch, daß der Fluoreszenz-markierte Antikörper nicht unmittelbar an den unbekannten Wirkstoff W_{X} bindet. Der unbekannte Wirkstoff W_{X} ruft vielmehr bei der Bindung an den Rezeptor R₁ eine Konformationsänderung im Rezeptor R₁ hervor (allosterischer Effekt). Durch diese Konformationsänderung werden am Rezeptor neue strukturelle Domänen freigelegt. An diese Domänen wird eine molekulare biologische Sonde, z.B. ein fluoreszenzmarkierter Antikörper (FITC) binden gelassen. So kann der neue Wirkstoff W_{X} indirekt durch die von ihm hervorgerufene Fluoreszenz nachgewiesen werden.

In vorstehend beschriebenen Fällen können die Wirkstoffe aus geeigneten Lösungsmitteln hinzugegeben werden, z.B. wäßrigen oder ethanolischen Salzlösungen oder geeigneten Lösungsmitteln. Die Messung kann selbst kann im Lösungsmittel oder an der getrockneten Probe erfolgen. Die Untersuchung der Wirkstoffe muß nicht einzeln erfolgen, sondern es können in gleicher Weise Mischungen von Wirkstoffen, auch Mischungen mit anderen Verbindungen, untersucht werden (vgl. Fig. 2). Eine Immobilisierung unter Benutzung von ELISA-Platten [96-Loch-Mikrotiterplatten] und entsprechenden Proben- und Lösungshandling-Systemen, d.h. etwa Pipettierautomaten für nanostrukturierte Systeme ist möglich. Natürlich können in gleicher Weise auch auf zellulären Strukturen fixierte Rezeptoren untersucht werden.

Die Immobilisierung des Moleküls - sei es Rezeptor, Ligand oder ein anderes Molekül z.B. Neurorezeptoren oder Rezeptoren auf Zellen des Immunsystems oder andere zelluläre Bindeproteine auf dem Substrat - kann kovalent oder nicht kovalent erfolgen. Eine nicht kovalente Immobilisierung erfolgt z.B. durch unmittelbare Adsorption. Die Immobilisierung kann jedoch auch über kovalente Bindungen erfolgen, wenn das Substrat eine entsprechende aktivierte Oberfläche aufweist. Beispielsweise kann bei der Flüssigphasenadsorption (Self-Assembly [S. Akari et al., Adv. Mater. 7 (1995) 549]) eine Aktivierung der Substratoberfläche dadurch erfolgen, daß eine Thiolcarbonsäure aufgebracht wird. Das Thiol bindet mittels Adsorption an das Substrat, z.B. eine Goldoberfläche, wobei die Säuregruppe nach außen weist.

Die Bindung des Moleküls A muß auch nicht direkt an die Substratoberfläche oder die Thiolcarbonsäure erfolgen. Sie kann auch indirekt sein. Beispielsweise kann zwischen der vorstehend genannten Thiolcarbonsäure und dem Molekül A, z.B. einem Protein, Rezeptor, Liganden usw. ein sog. Abstandshalter ["spacer"] angeordnet sein. Ein solcher Abstandshalter oder "spacer" ist Aminohexansäure oder Spermidin. Diese Moleküle können über ihre Aminogruppen an die vorstehend genannte Thiolcarbonsäure binden.

Danach erfolgt die Bindung des Moleküls, d.h. des Rezeptorwirkstoffs, Liganden o.ä. an die Substratoberfläche. Im vorstehend genanntem Falle erfolgt diese Bindung kovalent über den Abstandhalter.

Falls das Einfügen eines Abstandhalters zwischen der Substratoberfläche und dem zu untersuchenden Molekül A aus bestimmten Gründen, wie beispielsweise aufgrund von ungünstigen sterischen Konformationen noch nicht ausreichend ist, so kann der Schritt des Einfügens eines Abstandhalters auch mehrfach wiederholt werden. Beispielsweise können zwei oder mehr Moleküle des vorstehend genannten Abstandhalters Aminohexansäure aneinander gehängt werden. Eine Grenze in der Zahl der aneinander gehängten Abstandhalter ist erst dadurch gegeben, daß es bei einem zu großen Abstand zwischen der Oberfläche des Substrats und dem daran über den Abstandhalter zu bindenden Molekül A nicht mehr zu einer Immobilisierung im eigentlichen Sinne des Wortes kommt, da ein sehr langer Abstandhalter natürlich entsprechend flexibel ist und somit der Einsatz von SNOM unmöglich gemacht wird.

Aufgrund des Meßprinzips ist es ganz besonders vorteilhaft, daß eine Parallelisierung des Verfahrens möglich ist. Es können statt einer Lichtleiterfaser gleichzeitig mehrere Fasern verwendet werden. Der Nachweis der Fluoreszenz kann dann über Faserkoppler und/oder Detektorarrays erfolgen. Somit ist es möglich, mehrere Näpfchen z.B. einer ELISA-Platte oder gar die ganze Platte mit in der Regel 96 Nachweisstellen direkt auszulesen.

Dies erfolgt jedoch vorteilhafterweise mit der erfindungsgemäßen Einrichtung, umfassend eine Nahfeldmikroskopiervorrichtung, eine Einzel- oder Mehrfachprobenaufnahmevorrichtung und eine Steuereinrichtung zum Ansteuern der einzelnen Kompartimente der Einzel- oder Mehrfachprobenaufnahmevorrichtung mit der SNOM-Vorrichtung. Natürlich kann auch der Steuerungsvorgang umgekehrt ausgerichtet sein, d.h. mit der beweglichen Mehrfachprobenaufnahmevorrichtung läßt sich die Nachfeldmikroskopiervorrichtung ansteuern, ähnlich wie bei üblichen Mikroskopen der Objektisch bewegt wird.

Als Mehrfachprobenaufnahmevorrichtung weist diese Vorrichtung vorteilhafterweise eine Mehrloch-Mikrotiterplatte auf, wie sie z.B. auch bei ELISA-Tests verwendet wird. Vorzugsweise handelt es sich bei einer derartigen Mehrfachprobenaufnahmevorrichtung um eine solche, deren Abmessungen zwecks Erhöhung der Geschwindigkeit des erfindungsgemäßen Wirkstoff-Screening-Verfahrens miniaturisiert sind. In diesem Falle ist die Untergrenze für die Größe (Durchmesser) der einzelnen Kompartimente lediglich durch die Größe der Lichtleiterfaser festgelegt. Die Untergrenze liegt dadurch bei etwa 10 nm.

Nach oben hin gibt es grundsätzlich keine Begrenzung. In Hinsicht auf die Optimierung der Geschwindigkeit des Screening-Verfahrens ist es aber nicht sehr effektiv, wenn die Größe des Kompartiments bei der miniaturisierten Vorrichtung einige 100 µm, insbesondere 500 µm überschreitet. Eine bevorzugte obere Grenze liegt bei 150 µm.

Bei einer derartig miniaturisierten Aufnahmevorrichtung ist es für die Optimierung der Geschwindigkeit auch nicht mehr nötig, daß das Einzelkompartiment, das die Probe enthält, die Form eines Lochs ["well"] aufweist, wie sie von den üblichen Mikrotiterplatten bekannt ist. Als Strukturen können z.B. durch Lithographie und/oder Ätz- sowie Stempelprozesse hergestellte Vertiefungen, Näpfchen oder auch metallische Erhebungen z.B. in oder auf Silicium oder anderen Materialien wie z.B Kunststoffen oder Glas dienen. Denkbar ist auch die Verwendung von Aufdampfstrukturen oder solchen, die durch Self-Assembly-Verfahren hergestellt werden. Auch nach dem LIGA-Verfahren hergestellte Mikrostrukturen können verwendet werden.

Zum noch schnelleren Ablesen eines Kompartiments reicht es aus, wenn dieses die Form eines Napfs, Trogs oder einer flachen Vertiefung aufweist. Die in dieser Hinsicht vorteilhafteste Ausgestaltung der bei dem erfindungsgemäßen Verfahren bzw. der erfindungsgemäßen Einrichtung eingesetzten Mehrfachprobenaufnahmevorrichtung besteht einfach aus einer sehr flachen planaren Scheibe, auf der die zu untersuchenden Proben in Form von kleinen Tröpfchen aufgesetzt sind. Falls als Substrat ein Si-Wafer verwendet wird, auf den die Proben in Form von Tröpfchen aufgesetzt sind, ist es bevorzugt, wenn die betreffenden Stellen sich in ihrer Oberflächenstruktur von der Umgebung unterscheiden. Beispielsweise ist es im Falle, daß der Wafer mit Gold bedampfte Bereiche aufweist, bevorzugt, daß die einzelnen Kompartimente mit Gold unterlegt sind, während das hydrophobe Silicium die einzelnen Kompartimente voneinander abtrennt.

Die gesamte Einrichtung, umfassend die Nahfeldmikroskopiervorrichtung, die Einzel- oder Mehrfachprobenaufnahmevorrichtung mit Kompartimenten für Proben und die Steuereinrichtung, kann so modifiziert sein, daß die Steuereinrichtung statt der SNOM-Vorrichtung die Mehrfachprobenaufnahmevorrichtung steuert, ähnlich wie bei im Bereich der Lichtmikroskopie üblichen Mikroskopen der Objekttisch bewegt wird - wie bereits vorstehend erwähnt.

Der Steuerungsmechanismus selbst kann ähnlich dem erfolgen, wie er aus anderen computergesteuerten Vorrichtungen dem Fachmann vertraut ist, z.B. aus Schrittmotorsteuerungen.

Generell können die einzelnen Kompartimente einer derartigen Mehrfachprobenaufnahmevorrichtung beliebig angeordnet sein, insbesondere aber in Rechteck- oder Kreisstruktur.

### Beispiele

Nachfolgend wird ein Beispiel für das erfindungsgemäße Verfahren gegeben:
Für den Nachweis einer Fluoreszenz wurde ein kommerzielles SNOM-Gerät (z.B. Aurora; TopoMetrix Inc.) eingesetzt. Als Substrat diente eine mit Gold bedampfte ELISA-Platte oder ein Si-Wafer. Mittels der Flüssigphasenadsorption (Self-Assemby) wurde darauf eine Thiolcarbonsäure aufgebracht. Das Thiol bindet an die Goldoberfläche, die Säuregruppen weisen nach außen. Hieran wurde über eine Peptidbindung der relevante Rezeptor, z.B. Avidin oder Thrombin immobilisiert.

Dies erfolgte dadurch, daß man die mit Carboxylgruppen modifizierte Oberfläche oder Spitze mit einem vorstehend erwähnten Abstandhalter (z.B. Aminohexansäure oder Spermidin) oder einem anderen Liganden derivatisierte. Dazu wurden die Carboxylgruppen des Substrats oder der Goldspitze mit je 100 mM N-Hydroxysuccinimid (C₄H₅NO₃) und N-(3-Dimethylaminopropyl)-N'-ethylcarbodiimid(hydrochlorid) C₈H₁₈CIN₃ für 5 Minuten mit Aminohexansäure oder Spermidin, inkubiert (100 mM jeweils).

Die mit Aminohexansäure derivatisierte Oberfläche wurde danach erneut-wie oben beschrieben - mit N-Hydroxysuccinimid und N-(3-Dimethylamino-propyl)-N'-ethylcarbodiimid aktiviert. Danach wurde erneut mit dem aminogruppenhaltigen Ligand (z.B. Thrombin oder einem anderen Protein) in einer Konzentration von beispielsweise 100 µg/ml umgesetzt. Es können aber auch niedermolekulare Liganden wie z.B. folgender Thrombininhibitor an die Oberfläche gebunden werden. 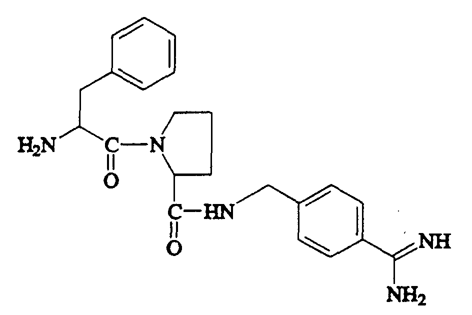 Wurde als Abstandhalter Spermidin gewählt, so kann man die freie Aminogruppe des Spermidins mit einer aktivierten Carboxylgruppe des Liganden umsetzen. Diese aktivierte Carboxylgruppe kann ebenfalls nach dem vorstehend beschriebenen Verfahren hergestellt werden. Es sind jedoch auch andere Verfahren einsetzbar, wie sie beispielsweise aus der Peptidsynthese zur Aktivierung von Carboxylgruppen bekannt sind:
a) Aktivierung der Carbonsäuren zum gemischten Anhydrid mit Chlorameisensäure, EEDQ, IIDQ
b) Aktivierung der Carbonsäure zum symmetrischen Anhydrid mit Carbodiimiden
c) Aktivierung als Aktivester z.B. mit Carbodiimid, Uroniumsalzen des Benztrazols oder Uroniumsalzen allgemein und eventuell Pentafluorphenol, Hydroxyssuccinimid, Hydroxybenzotriazol oder davon abgeleiteten Verbindungen
d) als Halogenide bzw. Pseudohalogenide z.B. als Fluorid mit TFF, als Bromid mit BrOP oder als Azid

Danach wurde fluoreszenzmarkiertes Avidin oder Thrombin hinzugegeben, das an den Referenzwirkstoff band. Folglich bildete sich ein fluoreszierender Komplex direkt an der Oberfläche des Substrats aus. Diese Einzelmolekül-Fluoreszenz konnte mittels SNOM nachgewiesen werden. Wurde nun eine Mischung mit einem effektiveren Wirkstoff hinzugegeben, so wurde das markierte Avidin bzw. Thrombin aus seiner Bindung befreit und diffundierte von der Oberfläche weg. Dadurch nahm die Fluoreszenz ab. Der in der zugegebenen Mischung enthaltene effektivere Wirkstoff muß aber nicht unbedingt an die gleiche Stelle binden wie der auf der Substratoberfläche immobilisierte Wirkstoff. Eine Verringerung der Bindung des mit Fluorescein markierten Moleküls kann auch durch Bindung an eine andere Stelle desselben erfolgen. Durch eine Beeinflussung der Konformation (allosterischer Effekt) wird dann die Affinität des fluoreszenzmarkierten Moleküls zu dem an der Substratoberfläche immobilisierten Liganden herabgesetzt, so daß nur noch ein geringerer Teil der fluoreszierenden Moleküle an der Oberfläche gebunden bleibt und somit die Fluoreszenz ebenfalls abnimmt.

Als Beispiel für zelluläre Strukturen, die als Substrat dienen können, seien insbesondere membranfixierte Rezeptoren genannt; neben den klassischen signaltransduzierenden Rezeptoren (z.B. G-Protein gekoppelten Rezeptoren, Liganden- und spannungsabhängigen Ionenkanälen) sind auch Rezeptoren auf Zellen des Immunssystems oder auf den Blutplättchen (z.B. GPIIb/IIIa Rezeptoren) sowie reine Bindeproteine auf Zellen wichtige zelluläre Ziele.

## Patentansprüche

1. Verfahren zum Wirkstoff-Screening, wobei man den Wirkstoff über die mittels einer rasteroptischen Nahfeldmikroskopiervorrichtung (Scanning Nearfield Optical Microscopy-Vorrichtung, SNOM-Vorrichtung) beobachtbare Änderung eines Signals [S], das durch einen optischen Kontrastmechanismus gekennzeichnet ist, nachweist.

2. Verfahren nach Anspruch 1, wobei das rasteroptische Nahfeld mittels einer Lichtleiterfaser (1) bereitgestellt wird.

3. Verfahren nach Anspruch 1 oder 2, wobei das Wirkstoffscreening die Schritte umfaßt, daß eine Mehrfachprobenaufnahmevorrichtung mit mehreren Kompartimenten für Proben bereitgestellt wird und an allen oder einem Teil dieser Kompartimente, bevorzugt mittels einer parallelisierten SNOM-Vorrichtung, die Schritte durchgeführt werden, daß
a) ein Molekül A an ein Substrat (2) direkt oder indirekt, kovalent oder nicht-kovalent gebunden wird,
b) ein mittels der rasteroptischen Nahfeldmikroskopiervorrichtung mittels eines Signals [S] nachweisbares Molekül B nicht-kovalent an das Molekül A binden gelassen wird,
c) eine einen Wirkstoff W2 enthaltende Lösung hinzugegeben wird und die Anwesenheit des Wirkstoffs W2 über die Änderung der Bindung des Moleküls B und damit des Signals [S] nachgewiesen wird.

4. Verfahren nach Anspruch 1 oder 2, wobei das Wirkstoff-Screening die Schritte umfaßt, daß eine Mehrfachprobenaufnahmevorrichtung mit mehreren Kompartimenten für Proben bereitgestellt wird und an allen oder einem Teil dieser Kompartimente - parallel oder nacheinander - die Schritte durchgeführt werden, daß
a) ein Molekül A an ein Substrat (2) binden gelassen wird,
b) eine einen Wirkstoff (Wx) enthaltende Lösung hinzugegeben wird,
c) der Wirkstoff (Wx) an A unter Bildung eines Molekülkomplexes A-X binden gelassen wird,
d) eine Lösung, die eine molekulare Sonde (FİTC) enthält, die über das Signal [S] mittels der rasteroptischen Nahfeldmikroskopiervorrichtung nachweisbar ist, zugegeben wird,
e) die molekulare Sonde FİTC an den Komplex A-X binden gelassen wird und
f) die Bindung von FİTC an A-X durch Messung von [S] nachgewiesen wird.

5. Verfahren nach einem der vorhergehenden Ansprüche, wobei es sich bei dem Signal [S] um ein Fluoreszenzsignal handelt.

6. Verfahren nach einem der vorhergehenden Ansprüche, wobei die Bindung des Moleküls A an das Substrat (2) mittels Adsorption erfolgt.

7. Verfahren nach einem der vorhergehenden Ansprüche, wobei es sich bei dem Substrat (2) um einen Si-Wafer, eine mit Au bedampfte Mikrotiterplatte, Glimmer, Graphit oder um eine zelluläre Struktur oder eine Kombination davon handelt.

8. Verfahren nach Anspruch 7, wobei es sich bei der zellulären Struktur um membranfixierte Rezeptoren handelt.

9. Verfahren nach einem der vorhergehenden Ansprüche, wobei das Molekül A an das Substrat (2) indirekt über einen Abstandhalter, insbesondere Spermidin oder Aminohexansäure gebunden wird.

10. Verfahren nach Anspruch 9, wobei der Abstandhalter seinerseits über eine an das Substrat (2) adsorbierte Verbindung gebunden wird.

11. Verfahren nach einem der vorhergehenden Ansprüche, wobei eine die SNOM-Vorrichtung umfassende parallelisierte Meßeinrichtung zum gleichzeitigen Nachweis mehrerer Probenkompartimente zum Wirkstoff-screening eingesetzt wird.

12. Verfahren nach einem der vorhergehenden Ansprüche, wobei das Signal [S] zum Nachweis der Bindung des Stoffs X einen optischen Kontrast verwendet, insbesondere Unterschiede in der Absorption, Fluoreszenz, Polarisation oder deren Wellenlängenabhängigkeit.

13. Verfahren nach einem der Ansprüche 10 bis 12, wobei der Schritt des Bindens des Abstandhalters mehrfach wiederholt wird.

14. Verwendung einer rasteroptischen Nahfeldmikroskopiervorrichtung (SNOM-Vorrichtung) zum Wirkstoffscreening, wobei man den Wirkstoff über die mittels der rasteroptischen Nahfeldmikroskopiervorrichtung beobachtbare Änderung eines Signals [S] nachweist.

15. Verwendung nach Anspruch 14, wobei man eines der Verfahren nach Anspruch 1 bis 13 durchführt.

16. Einrichtung zum Wirkstoffscreening, umfassend eine Nahfeldmikroskopiervorrichtung, eine Einzel- oder Mehrfachprobenaufnahmevorrichtung mit Kompartimenten für Proben und eine Steuereinrichtung zum Ansteuern der einzelnen Kompartimente der Einzel- oder Mehrfachprobenaufnahmevorrichtung mit der Nahfeldmikroskopiervorrichtung.

17. Einrichtung zum Wirkstoffscreening, umfassend eine Nahfeldmikroskopiervorrichtung, eine Ein-zel- oder Mehrfachprobenaufnahmevorrichtung mit Kompartimenten für Proben und eine Steuereinrichtung zum Ansteuern der Nahfeldmikroskopiervorrichtung mit der Einzel- oder Mehrfachprobenaufnahmevorrichtung.

18. Einrichtung nach einem der vorhergehenden Ansprüche, wobei es sich bei der Einzel- oder Mehrfachprobenaufnahmevorrichtung um eine Mehrfachloch-Mikrotiterplatte handelt.

19. Mehrfach-Nanotitervorrrichtung zum Wirkstoffscreening, umfassend eine planare Platte sowie mehrere darauf angeordnete Nano-Kompartimente,
wobei die Größe der Kompartimente (Durchmesser) 10 bis 100 nm beträgt.

20. Mehrfach-Nanotitervorrichtung nach Anspruch 19, wobei die Nano-Kompartimente in der Form napfartiger Vertiefungen vorliegen.

21. Mehrfach-Nanotitervorrichtung nach Anspruch 19, wobei die Nano-Kompartimente von den übrigen Bereichen der planaren Platte dadurch abgegrenzt sind, daß ihre Größe (Durchmesser) durch eine von den letzteren sich unterscheidende andersartige chemische Struktur oder Zusammensetzung festgelegt ist.

22. Mehrfach-Nanotitervorrichtung nach einem der Ansprüche 19 bis 21, wobei es sich bei der planaren Platte um hydrophobes Silicium handelt und die Nano-Kompartimente durch eine Veränderung der Oberflächenstruktur festgelegt sind.

## Claims

1. A method of screening active compounds, in which the active compound is detected from the change in a signal [S] which can be observed by means of a scanning near field optical microscopy instrument (SNOM instrument), which signal is characterized by an optical contrast mechanism.

2. A method as claimed in claim 1, in which the scanning optical near field is provided by means of an optical fiber (1).

3. A method as claimed in claim 1 or 2, in which the active compound screening comprises the steps of providing a multiple sample holder with a plurality of compartments for samples, and carrying out the following steps on all or some of these compartments, preferably by means of a parallelized SNOM instrument:
a) a molecule A is bound directly or indirectly, covalently or noncovalently, to a substrate (2),
b) a molecule B which can be detected from a signal [S] by means of a scanning near field optical microscopy instrument is allowed to bind noncovalently to the molecule B,
c) a solution containing an active compound W2 is added, the presence of the active compound W2 is detected from the change in binding of the molecule B and thus of the signal [S].

4. A method as claimed in claim 1 or 2, in which the active compound screening comprises the steps of providing a multiple sample holder having a plurality of compartments for samples, and carrying out the following steps on all or some of these compartments - in parallel or successively:
a) a molecule A is allowed to bind to a substrate (2),
b) a solution containing an active compound (Wx) is added,
c) the active compound (Wx) is allowed to bind to A with formation of a molecule complex A-X,
d) a solution containing a molecular probe (FiTC) which can be detected from the signal [S] by means of the scanning near field optical microscopy instrument is added,
e) the molecular probe (FiTC) is allowed to bind to the complex A-X, and
f) the binding of (FiTC) to A-X is detected by measurement of [S].

5. A method as claimed in any one of the preceding claims, where the signal [S] is a fluorescence signal.

6. A method as claimed in any one of the preceding claims, where the binding of the molecule A to the substrate (2) takes place by adsorption.

7. A method as claimed in any one of the preceding claims, where the substrate (2) is an Si wafer, a microtitration plate with a vapor-deposited Au coating, mica, graphite or a cellular structure, or a combination tnereof.

8. A method as claimed in claim 7, where the cellular structure is a membrane-immobilized receptor.

9. A method as claimed in any one of the preceding claims, where the molecule A is bound indirectly to the substrate (2) via a spacer, in particular spermidin or aminohexanoic acid.

10. A method as claimed in claim 9, where the spacer is itself bound via a compound adsorbed onto the substrate (2).

11. A method as claimed in any one of the preceding claims, where a parallelized measurement device which includes the SNOM instrument is employed for simultaneous detection of a plurality of sample compartments for active compound screening.

12. A method as claimed in any one of the preceding claims, where the signal [S] uses an optical contrast, in particular a difference in absorption, fluorescence, polarization or wavelength dependence thereof, for detecting the binding of substance X.

13. A method as claimed in any one of claims 10 to 12, where the spacer binding step is repeated a number of times.

14. The use of a scanning near field optical microscopy instrument (SNOM instrument) for screening active compounds, where the active compound is detected from the change in a signal [S] which can be observed by means of the scanning near field optical microscopy instrument.

15. The use as claimed in claim 14, in which one of the methods as claimed in claims 1 to 13 is carried out.

16. An apparatus for active compound screening comprising a near field microscopy instrument, a single or multiple sample holder having compartments for samples, and a control device for addressing the individual compartments of the single or multiple sample holder with the near field microscopy instrument.

17. An apparatus for active compound screening comprising a near field microscopy instrument, a single or multiple sample holder having compartments for samples, and a control device for addressing the near field microscopy instrument by means of the single or multiple sample holder.

18. An apparatus as claimed in any one of the preceding claims, where the single or multiple sample holder is a multiwell microtitration plate.

19. A multiple nanotitration instrument for active compound screening comprising a planar plate and a plurality of nanocompartments arranged thereon, where the size (diameter) of the compartments is from 10 to 100 nm.

20. A multiple nanotitration instrument as claimed in claim 19, where the nanocompartments are in the form of wells.

21. A multiple nanotitration instrument as claimed in claim 19, where the nanocompartments are delimited from the remaining areas of the planar plate in that their size (diameter) is determined by a chemical structure or composition which is different from that of these remaining areas.

22. A multiple nanotitration instrument as claimed in any one of claims 19 to 21, where the planar plate is hydrophobic silicon, and the nanocompartments are defined by a change in the surface structure.

## Revendications

1. Procédé pour la recherche de substances actives dans lequel on décèle la substance active par la variation d'un signal [S] caractérisé par un mécanisme de contraste optique, observable à l'aide d'un dispositif de microscopie optique à réseau à champ rapproché (dispositif de Scanning Nearfield Optical Microscopy, "SNOM").

2. Procédé selon la revendication 1, dans lequel le champ optique à réseau rapproché est produit à l'aide d'une fibre conductrice de lumière (1).

3. Procédé selon la revendication 1 ou 2, dans lequel la recherche de substances actives comprend l'utilisation d'un dispositif à prélèvement d'échantillons multiples à plusieurs compartiments sur la totalité ou une partie desquels, de préférence à l'aide d'un dispositif SNOM parallélisé, on procède aux opérations suivantes :
a) on fixe directement ou indirectement, par liaisons covalentes ou non-covalentes, une molécule A sur un substrat (2),
b) on provoque la fixation sur la molécule A, par des liaisons non-covalentes, d'une molécule B décelable par un signal S à l'aide du dispositif de microscopie optique à réseau à champ rapproché,
c) on ajoute une solution contenant une substance active W2 et on décèle la présence de la substance active W2 par la modification de la fixation de la molécule B et, par suite, du signal [S].

4. Procédé selon la revendication 1 ou 2, dans lequel la recherche de substances actives comprend l'utilisation d'un dispositif à prélèvement d'échantillons multiples à plusieurs compartiments sur la totalité ou une partie desquels - parallèlement ou successivement - on procède aux opérations suivantes :
a) on provoque la fixation d'une molécule A sur un substrat (2),
b) on ajoute une solution contenant une substance active (Wx),
c) on provoque la fixation de la substance active (Wx) sur A avec formation d'un complexe moléculaire A-X,
d) on ajoute une solution contenant une sonde moléculaire (FITC) décelable par le signal [S] à l'aide du dispositif de microscopie optique à réseau à champ rapproché,
e) on provoque la fixation de la sonde moléculaire FITC sur le complexe A-X et
f) on met en évidence la fixation de FITC sur A-X par mesure de [S].

5. Procédé selon une des revendications qui précèdent, dans lequel le signal [S] est un signal de fluorescence.

6. Procédé selon l'une des revendications qui précèdent, dans lequel la fixation de la molécule A sur le substrat (2) est réalisée par adsorption.

7. Procédé selon l'une des revendications qui précèdent dans lequel le substrat (2) est une puce de silicium, une plaque à microtitrage dorée par déposition de vapeurs, du mica, du graphite ou une structure cellulaire ou une combinaison de ces substrats.

8. Procédé selon la revendication 7, dans lequel la structure cellulaire consiste en récepteurs fixés sur membranes.

9. Procédé selon l'une des revendications qui précèdent, dans lequel la molécule A est fixée sur le substrat (2) de manière indirecte, par l'intermédiaire d'un espaceur consistant en particulier en spermidine ou acide aminohexanoïque.

10. Procédé selon la revendication 9, dans lequel l'espaceur est lui-même fixé sur un composé adsorbé par le substrat (2).

11. Procédé selon une des revendications qui précèdent, dans lequel on utilise pour la recherche de substances actives, un dispositif de mesure parallélisé comprenant le dispositif SNOM pour l'examen simultané de plusieurs compartiments à échantillons.

12. Procédé selon une des revendications qui précèdent, dans lequel le signal [S] pour mise en évidence de la fixation de la substance X exploite un contraste optique, en particulier des différences dans l'absorption, la fluorescence, la polarisation ou la variation en fonction de la longueur d'ondes.

13. Procédé selon une des revendications 10 à 12, dans lequel l'opération de fixation de l'espaceur est répétée plusieurs fois.

14. Utilisation d'un dispositif de microscopie optique à réseau à champ rapproché (dispositif SNOM) pour la recherche de substances actives, dans lequel on décèle la substance active par la modification d'un signal [S] observable à l'aide du dispositif de microscopie optique à réseau à champ rapproché.

15. Utilisation selon la revendication 14, dans laquelle on exploite l'un des procédés des revendications 1 à 13.

16. Dispositif pour la recherche de substances actives comprenant un dispositif de microscopie à champ rapproché, un dispositif à prélèvement d'échantillon individuel ou d'échantillons multiples avec des compartiments pour les échantillons et un dispositif de commande permettant de diriger le dispositif de microscopie à champ rapproché sur les divers compartiments du dispositif de prélèvement d'échantillon individuel ou d'échantillons multiples.

17. Dispositif pour la recherche de substances actives, comprenant un dispositif de microscopie à champ rapproché, un dispositif à prélèvement d'échantillon individuel ou d'échantillons multiples avec des compartiments pour les échantillons et un dispositif de commande permettant de diriger le dispositif de microscopie à champ rapproché vers le dispositif à prélèvement d'échantillon individuel ou d'échantillons multiples.

18. Dispositif selon une des revendications qui précèdent, dans lequel le dispositif de prélèvement d'échantillon individuel ou d'échantillons multiples est une plaque à microtitrage à plusieurs orifices.

19. Dispositif de nanotitrage multiple pour la recherche de substances actives, comprenant une plaque plane portant plusieurs nanocompartiments, la dimension des compartiments (diamètre) allant de 10 à 100 nm.

20. Dispositif de nanotitrage multiple selon la revendication 19, dans lequel les nanocompartiments ont la forme de godets.

21. Dispositif de nanotitrage multiple selon la revendication 19, dans lequel les nanocompartiments sont limités par les autres régions de la plaque plane, leur dimension (diamètre) étant déterminée par une structure ou composition chimique différente de celle de ces dernières.

22. Dispositif de nanotitrage multiple selon l'une des revendications 19 à 21, dans lequel la plaque plane consiste en silicium hydrophobe et les nanocompartiments sont formés par une modification de la structure superficielle.
